Europäisches Patentamt

European Patent Office

Office européen des brevets

(19)

(11)  **EP 1 237 530 B1**

(12)  **EUROPEAN PATENT SPECIFICATION**

(45) Date of publication and mention
of the grant of the patent:
**30.06.2004  Bulletin 2004/27**

(21) Application number: **00979786.1**

(22) Date of filing: **30.11.2000**

(51) Int Cl.⁷: **A61K 7/135**, A61K 7/13

(86) International application number:
**PCT/GB2000/004555**

(87) International publication number:
**WO 2001/043709 (21.06.2001 Gazette 2001/25)**

(54) **HAIR BLEACHING AND COLOURING COMPOSITIONS HAVING A PH GREATER THAN PH 10 AND COMPRISING CHOLESTEROL**

HAARBLEICHENDE UND HAARFÄRBENDE ZUBEREITUNG MIT EINEM PH GRÖSSER ALS PH 10 UND CHOLESTEROL ENTHALTEND

COMPOSITIONS DE COLORATION ET DE DECOLORATION DES CHEVEUX AYANT UN PH SUPERIEUR A 10 ET CONTENANT DU CHOLESTEROL

(84) Designated Contracting States:
**AT BE CH CY DE DK ES FI FR GB GR IE IT LI LU MC NL PT SE TR**

(30) Priority:  **17.12.1999 EP 99310219**

(43) Date of publication of application:
**11.09.2002  Bulletin 2002/37**

(73) Proprietors:
• **UNILEVER PLC
London EC4P 4BQ (GB)**
Designated Contracting States:
**CY GB IE**
• **UNILEVER N.V.
3013 AL Rotterdam (NL)**
Designated Contracting States:
**AT BE CH LI DE DK ES FI FR GR IT LU MC NL PT SE TR**

(72) Inventors:
• **MADDEN, Timothy, John,
Unilever Res. Port Sunlight
Wirral, Merseyside CH63 3JW (GB)**

• **VRETTOU, Christina, Unilever Res. Port Sunlight
Wirral, Merseyside CH63 3JW (GB)**
• **BROWNBILL, Susan, Unilever Res. Port Sunlight
Wirral, Merseyside CH63 3JW (GB)**
• **PEARCE, Matthew L.,
Unilever Res. Port Sunlight
Wirral, Merseyside CH63 3JW (GB)**

(74) Representative: **Tansley, Sally Elizabeth et al
Unilever PLC,
Patent Division,
Colworth House
Sharnbrook, Bedford MK44 1LQ (GB)**

(56) References cited:
**EP-A- 0 065 718          CH-A- 272 709
DE-A- 4 005 008          DE-A- 4 017 718
FR-A- 2 051 629          GB-A- 466 172
GB-A- 486 086            GB-A- 653 053
GB-A- 926 977            GB-A- 2 033 939
US-A- 5 021 066**

## Description

### FIELD OF THE INVENTION

This invention relates to hair bleaching and colouring compositions and methods of bleaching and colouring hair.

### BACKGROUND OF THE INVENTION

Hair contains the pigment melanin in granular form within the hair cortex. The darker the perceived hair colour, the higher the concentration of melanin in the hair. Chemical and natural dyes may also be applied to hair to alter the perceived hair colour. The objective of hair bleaching compositions is to decolourise both the naturally occurring. melanin pigment and in addition any synthetic pigments applied to the hair. Hair colouring compositions generally contain agents first to decolourise the hair and dyes subsequently to colour the decolourised hair.

Hair bleaching and colouring treatment of hair is generally associated with a number of disadvantages such as causing hair brittleness and damage.

Damage to the hair typically manifests itself in cuticle and protein loss from the hair fibre, excessive fibre stiffness, hair fibre brittleness and breakage and frayed or split ends.

Solutions to the problem of hair damage caused by hair bleaching and colouring compositions have been suggested in the art. WO 98/27945 describes the use of a low pH (pH 1 to 6) oxidative hair dye system to reduce hair damage. JP 63-297317 describes the optional use of cholesterol in a hair dye composition to improve the strength and feel of hair.

In general, the darker the hair the higher the pH required in order to effectively decolourise the higher concentration of melanin pigment. Unfortunately these necessarily harsh conditions in order to deliver effective bleaching and colouring can be particularly damaging to the hair.

Thusfar hair bleaching and colouring compositions have been unable to fully satisfy the desire for effective bleaching with minimal hair damage, particularly for dark-brown and black hair.

Hair bleaching and/or colouring compositions which can deliver effective hair bleaching and/or colouring, even on dark hair, but which avoid or reduce hair damage and deliver improved damage repair and protection to the hair thus continue to be sought.

We have now found that if hair bleaching and colouring compositions are buffered to a pH greater than or equal to pH 10.3, the presence of cholesterol reduces damage, protects hair from damage and/or improves damage repair to a greater extent than in such compositions having a pH below pH 10. Such compositions are advantageous since they can deliver effective bleaching and colouring of dark hair whilst the damage generally caused by such high pH systems is reduced by a surprising extent.

Hair bleaching and colouring compositions according to the present invention demonstrate improved efficacy in the repair and prevention of the principal symptoms of damaged hair.

### SUMMARY OF THE INVENTION

According to a first aspect of the present invention there is provided a hair bleaching composition comprising:

(a) a peroxygen compound;
(b) a buffering agent; and
(c) cholesterol and/or derivatives thereof or mixtures thereof;

characterised in that the pH of the composition is greater than or equal to pH 10.3.

According to a second aspect of the present invention there is provided a hair colouring composition comprising the hair bleaching composition according to the first aspect and additionally a hair colouring agent.

According to a third aspect of the present invention there is provided a method of bleaching and/or colouring hair comprising applying to the hair the bleaching composition and/or colouring composition according to the first and/or second aspects of the invention.

According to a further aspect of the present invention there is provided a hair bleaching kit comprising:

(a) a first package containing a peroxygen compound;
(b) a second package containing a buffering agent; and
(c) cholesterol and/or derivatives thereof or mixtures thereof contained in the first and/or second package and/or in a third package;

characterised in that the peroxyen compound, buffering agent and cholesterol and/or derivatives thereof or mixtures thereof form, when mixed, a hair bleaching composition according to the first aspect of the present invention having a pH greater than or equal to 10.3

According to a still further aspect of the present invention there is provided a hair colouring kit comprising:

(a) a first package containing a peroxygen compound;
(b) a second package containing a buffering agent;
(c) cholesterol and/or derivatives thereof or mixtures thereof contained in the first and/or second package and/or in a third package; and
(d) a hair colouring agent contained in the second package and/or third package, and/or a fourth package;

characterised in that the peroxygen compound, buffering agent, hair colouring agent and cholesterol and/or derivatives thereof or mixtures thereof form, when mixed, a hair colouring composition according to the second aspect of the invention having a pH greater than or equal to 10.3.

A still further aspect of the present invention provides a method of bleaching and/or colouring hair using the hair bleaching and/or colouring kit described above comprising the steps of admixing the contents of the packages followed by applying the bleaching and/or colouring composition to the hair.

## DETAILED DESCRIPTION

### PEROXYGEN COMPOUND

The compositions of the invention comprise as an essential feature at least one peroxygen compound. Mixtures of two or more such compounds may be used if desired. The peroxygen compounds bleach hair by solubilising and oxidising melanin.

Hydrogen peroxide is the most preferred peroxygen compound for use in the bleaching and colouring compositions of the present invention, though many other peroxygen compounds are suitable.

Other peroxygen compounds suitable for use are generally water soluble peroxygen compounds such as peroxygen bleaches capable of yielding hydrogen peroxide in aqueous solution. Water soluble in this respect means a peroxygen compound which can be substantially solubilised in water at 25°C. Water soluble peroxygen bleaching compounds are well known in the art and, other than hydrogen peroxide, include inorganic alkali metal peroxides such as sodium priodate and sodium peroxide, organic peroxides such as urea peroxide and melamine peroxide and inorganic perhydrate salt bleaching compounds such as the alkali metal salts of perborates, percarbonates, perphosphates, persilicates, and persulphates.

The peroxygen compound is generally present in the compositions of the present invention at a level from 0.01% to 40%, preferably from 0.1% to 20%, most preferably from 1% to 7%, by weight of the composition.

### BUFFERING AGENT AND pH OF THE COMPOSITIONS

The pH of the bleaching and colouring compositions of the present invention are buffered such that they are greater than or equal to pH 10.3. A wide variety of buffering agents well known in the art are suitable for this purpose.

The most preferred buffering agent is an aqueous alkaline solution containing ammonia (ammonium hydroxide). Ammonia has the advantage of enhancing the bleaching and colouring treatment of hair by swelling the hair fibre to aid diffusion of peroxygen compounds into the hair. This allows faster and more effective solubilisation and decolouration of melanin or synthetic hair dyes.

Other suitable buffering agents include ethylamine, dipropylamine, triethylamine and alkanediamines such as 1,3-diaminopropane, anhydrous alkaline alkanolamines such as, mono or di- ethanolamine, hydroxides of alkali metals, such as sodium and potassium hydroxide, hydroxides of alkali earth metals, such as magnesium and calcium hydroxide, basic amino acids such as L-alginine, lysine, oxylysine and histidine and alkanolamines such as dimethylaminoethanol and aminoalkylpropanediol and mixtures thereof.

It is an essential feature of the present invention that the compositions are buffered to a pH which is greater than or equal to pH 10.3. In a preferred embodiment the pH of the compositions are buffered to a pH which is greater than or equal to pH 10.5.

A sufficient amount of buffering agent is present in the compositions of the present invention in order to buffer the compositions to the required pH in conventional manner.

We have now found that in hair bleaching and colouring compositions which are buffered to a pH greater than or equal to pH 10.3, cholesterol reduces damage, protects hair from damage and/or improves damage repair to a greater extent than in such compositions having a pH below pH 10. Such compositions are advantageous since they can deliver effective bleaching and colouring of dark hair whilst the damage generally caused by such high pH systems is reduced by a surprising extent.

CHOLESTEROL

A further essential component of the bleaching and colouring compositions of the present invention is cholesterol. Derivatives of cholesterol such as cholesterol esters, cholesterol fatty acid esters, and cholesterol acetate esters are also suitable for use in the compositions of the present invention.

The level of cholesterol or derivatives in the composition is suitably from 0.005% to 5%, preferably from 0.001% to 3%, optimally from 0.01% to 0.1%, by weight based on the total weight of the composition.

HAIR COLOURING AGENTS

The hair colouring compositions of the present invention additionally include one or more hair colouring agents. Hair colouring agents suitable for use in the compositions of the present invention include both oxidative and non-oxidative dyes and mixtures thereof. Such hair colouring agents may be used with the bleaching systems of the present invention to formulate permanent, demi-permanent, semi-permanent or temporary hair dye compositions.

(i) Oxidative Dyes

The dye forming intermediates used in oxidative dyes are generally classified as primary and secondary intermediates.

Primary intermediates are chemical compounds which by themselves will form a dye upon oxidation. The secondary intermediates, also known as colour modifiers or couplers are used with other intermediates for specific colour effects or to stabilise the colour.

Primary oxidative dye intermediates are generally colourless molecules prior to oxidation. The oxidative dye colour is generated when the primary intermediate is oxidised and subsequently enjoined with a secondary intermediate (coupling agent), which is also generally colourless, to form a coloured, conjugated molecule.

Oxidative dye intermediates diffuse into the hair shaft, and then are oxidised and coupled to form larger dye complexes within the hair shaft which are less readily washed out thus forming a "permanent" hair colour change.

The secondary intermediates (colour modifiers or couplers), are preferably used in conjunction with the oxidative dye precursors and are thought to interpose themselves in the coloured polymers during their formation and to cause shifts in the electronic spectra thereof, thereby resulting in slight colour changes.

A wide range of oxidative dyes suitable for use herein are well known in the art, such as aromatic diamines, amino phenols, polyhydric phenols and derivatives thereof. Preferred oxidative dyes for use in the present invention include m-phenylenediamine, toluene-2,5-diamine, p-phenylenediamine, resorcinol, o-aminophenol, m-aminophenol, p-aminophenol, 1-naphthol, 4-amino-2-hydroxytoluene, 2-methylresorcinol, N4, N4-bishydroxyethyl-p-phenylene diamine sulphate, 3-methyl-4-aminophenol, 1-phenyl-3-methyl-pyrazolone and 2-methyl-5-hydroxyethylaminophenol.

(ii) Non-oxidative Dyes

Non-oxidative dyes include direct dyes, semi-permanent, temporary and other dyes. Various types of non-oxidative dyes are well known in the art.

Natural dyes and vegetable dyes may also be included in the colouring compositions of the present invention. Examples include henna, camomile, indigo, logwood and walnut hull extract.

Temporary hair dyes, or hair colouring rinses, are generally comprised of dye molecules which are too large to diffuse into the hair shaft and which act on the exterior of the hair. They are usually applied via a leave- in procedure in which the dye solution is allowed to dry on the hair surface.

Semi-permanent hair dyes are generally larger than permanent (oxidative) dyes. Typically, semi-permanent dyes act in a similar manner to oxidative dyes in that they have the potential to diffuse into the hair shaft. However, semi-permanent dyes are generally smaller in size than the aforementioned conjugated oxidative dye molecules and as such are pre-disposed to gradual diffusion out of the hair again. Simple hair washing and cleaning action will encourage this process. Suitable semi-permanent dyes for use in the compositions of the present invention include HC Blue 2, HC Yellow 4, HC Red 3, Disperse Violet 4, Disperse Black 9, HC Blue 7, HC Yellow 2, Disperse Blue 3, Disperse violet I and mixtures thereof.

Direct dyes, such as nitro dyes do not require oxidation to dye the hair. They are usually applied to the hair in a base formulation which includes surfactant material. Suitable direct dyes include derivatives of nitroamino benzene, nitro aryl amines or azo dyes.

OPTIONAL MATERIALS AND COSMETIC ADJUNCT

Besides the actives, the compositions of the present invention may also contain other ingredients conventionally used in the art such as diluents, sequestrants, thickeners, carriers, surfactants (anionic, cationic, nonionic, amphoteric, zwitterionic and mixtures thereof), antioxidants, proteins, polypeptides, preservatives, moisturising agents, solvents, perfumes, enzymes, polymers and conditioners.

PRODUCT FORM

The compositions according to the present invention may be presented in kit form comprising a number of separate compartments containing the various ingredients of the final composition for mixing by the user immediately prior to application to the hair. The kit may comprise multiple containers or a single container having multiple compartments.

One component of the kit comprises an individually packaged peroxygen compound. A further kit component comprises an individually packaged buffering agent. In one embodiment of the present invention the individually packaged peroxygen compound comprises a stabilised aqueous solution of a watersoluble peroxygen bleach compound, most generally hydrogen peroxide in an amount such that the final concentration of the compound for use on the hair is from 0.5% to 14% by weight of the composition, and the individually packaged buffering agent comprises ammonium hydroxide in an amount such that the pH of the composition once mixed is greater than or equal to pH 10.3. The cholesterol (or derivatives) may be contained in the peroxygen compound compartment or the buffering agent compartment, or indeed in a further separate compartment. Optionally a hair colouring agent (for hair colouring compositions)and/or additional agents as herein before described may also be included in with the buffering agent compartment, cholesterol compartment (if present) or indeed in a still further separate compartment.

Most conveniently the kit comprises two separate compartments, one containing the peroxygen compound, the other containing the buffering agent and hair colouring agent (if present) and the other optional ingredients mentioned above; the cholesterol being present in either or both of those two compartments.

The compartmentalised ingredients of the composition are generally mixed by the user immediately prior to application to the hair.

METHOD OF USE

The compositions of the present invention may be used to bleach hair or may, in combination with hair colouring agents , be used to bleach and colour hair.

The bleaching/colouring compositions herein are applied to the hair for periods of from 1 minute to 90 minutes depending upon the degree of bleaching/colouring required. A preferred time is between 5 minutes and 30 minutes.

The products provide excellent hair bleaching and/or hair colouring together with minimal hair damage.

**EXAMPLES**

**Example 1**

This example demonstrates the hair damage reduction provided by cholesterol in hair bleaching compositions at various pHs.

**Step 1:Preparation of the Test bleaching Solutions.**

Two bleaching solutions, Solution 1 without cholesterol (as a control) and Solution 2 with cholesterol, were prepared in the following manner:

Bleaching Solution 1 (No Cholesterol)

| Material | %Active Species | %w/w | Amount (g) |
| --- | --- | --- | --- |
| SLES 2EO (Tegobetain CK) | 25.94 | 8.00 | 30.84 |
| CAPB (Tegobetain CK) | 30 | 4.00 | 13.33 |

(continued)

| Material | %Active Species | %w/w | Amount (g) |
|---|---|---|---|
| Dequest | 100 | 0.20 | 0.20 |
| Hydrogen Peroxide (ex Sigma) | 30 | 4.00 | 13.33 |
| **Water** (DI) | | | 42.29 |
| | | **Total** | 100.00 |

Bleaching Solution 2 (With Cholesterol)

| Material | %Active Species | %w/w | Amount (g) |
|---|---|---|---|
| SLES 2EO (Tegobetain CK) | 25.94 | 8.00 | 30.84 |
| CAPB (Tegobetain CK) | 30 | 4.00 | 13.33 |
| Cholesterol (ex Sigma)[a] | 100 | 0.01 | 0.01 - |
| Dequest | 100 | 0.20 | 0.20 |
| Hydrogen Peroxide (ex Sigma) | 30 | 4.00 | 13.33 |
| **Water** (DI) | | | 42.28 |
| | | **Total** | 100.00 |

For the bleaching solution with cholesterol, Solution 2, first the Dequest was weighted into the water in a tall form beaker and allowed to dissolve. Once dissolved the cholesterol and a small amount of SLES 2EO (approximately 1g) were weighted into the Dequest solution and heated in a waterbath to 70°C with continuous stirring using the small cog of a heidolph stirrer. The solution was left to stir and cool to approximately 35-40°C for at least an hour. Once cooled the solution was mixed using a high sheer mixer (Ystral) for 1 min on setting 4.

The solution was then returned to the heidolph and allowed to cool to room temperature with constant stirring. Once cool the CAPB and remaining SLES 2EO were added and mixed using the heidolph.

To prepare solution 1 (no cholesterol) there is no need for heating up the solution. The Dequest was weighted into water and then, once dissolved the CAPB was added and mixed using the heidolph. Once dissolved the SLES 2E0 was added and returned to the heidolph.

**Step 2: Bleaching Method**

The bleaching solutions 1 and 2 were each divided into three parts A, B and C. A was adjusted to pH 9.7, B to pH 10.1 and C to pH 10.5. Each solution was adjusted to the required pH with ammonium hydroxide (ex sigma, 28.1% active).

The six solutions were each dosed (1.6 ml/g hair) onto a separate Spanish hair switch (8g, 8", ex Hugo Royer Hair Suppliers) in a disposable petri dish using an automatic pipette. Each switch was then agitated for 90sec using a spoon ended plastic spatula. The switches were then left for 20 min. 1.6 ml/g hair of warm water was then pipetted onto each switch and they were agitated again for lmin. Each switch was then rinsed and left to dry naturally.

**Step 3: Cuticle Abrasion Method**

This method provides a measure of the extent of hair damage following the bleaching step.

First the switches from step 2 were washed for 30sec with 0.1ml/g hair of base wash. Rinse and repeat. The base wash used is 12% SLES, 2% CAPB in water. Dry them overnight. Cut the hair about lcm long throughout the whole length. Weight out 4g of the hair and place into the blender (Waring 11 commercial blender). Record the initial weight of the hair. Add 200ml of distilled water to the blender, making sure that the hair is covered with water. Blend hair in the water for 1 min. Transfer the liquid to a fresh tube. Wash the blender and add this, to the tube, as well. Pour the supernatant in 4 x 50ml centrifuge tubes and centrifuge at 4300rpm for 10mins, 22°C. Pour the pellet on the filters, wash the tube and add this as well. Transfer the supernatant to centrifuge tubes and centrifuge at 4300rpm for 10 min. Transfer the pellet to a beaker, wash the tube 2-3 times and top with water until 50ml. Freeze them until solid. Freeze dry them for 24 hours in Modulyo freeze drier. Once dry, weigh amount of cuticle recovered.

Final measure is calculated as follows:

$$((\text{weight of cuticle recovered})/(\text{initial hair weight}))*100$$

This measure provides the % weight of cuticle abraded from the hair fibres which reflects the state of damage of the hair tested. Thus the greater amount of cuticle recovered the more damaged the tested hair.

## RESULTS

Table 1

| pH | % Weight of cuticle recovered from hair bleached with solution 1 (cholesterol absent) | % Weight of cuticle recovered from hair bleached with solution 2 (cholesterol present) | % reduction in damage of hair when cholesterol present |
|---|---|---|---|
| 9.7* | 0.041 | 0.039 | 3.95 |
| 10.1* | 0.048 | 0.042 | 12.211 |
| 10.5 | 0.122 | 0.084 | 30.902 |

* Comparative example

From the results in Table 1 is it clear that the presence of Cholesterol reduces the damage caused to hair by bleaching since the % weight of cuticle recovered when Cholesterol is present is less than when Cholesterol is absent.

Surprisingly, however, the same amount of Cholesterol reduces the damage to hair by a significantly greater extent when the bleaching solution is buffered to a pH above 10.3 than below pH 10.3. For example, it can be seen that at pH 9.7 there is 3.95% less damage to the hair when Cholesterol is present, but surprisingly there is 30.902% less damage to the hair when the same amount of Cholesterol is present, but the bleaching solution is buffered to pH 10.5.

Thus we have surprisingly found that in a bleaching composition buffered to a pH greater than or equal to pH 10.3 cholesterol reduces hair damage to a greater extent than in a bleaching composition buffered to a pH below 10.

## Example 2

## Hair Bleaching Composition

A hair bleaching composition was prepared in conventional manner according to the formulation details below. The developer and base were stored in separate bottles before use. To use, the developer and base are mixed together to form a hair bleaching composition having a pH greater than pH10. The composition is applied to hair and left for 20 minutes. The hair is bleached effectively with minimal damage.

| Developer | | | |
|---|---|---|---|
| **Material** | **% Active Species** | **%w/t** | **Amount (g)** |
| Lowenol 6559 (ex. Lowenstein) | 100 | 1.0 | 1.0 |
| Phosphoric Acid (ex Sigma) | 85 | 0.03 | 0.03 |
| Hydrogen Peroxide (ex. Fisher) | 35 | 17.1 | 17.1 |
| Polyquaternium 37 (ex Ciba) | 100 | 1.0 | 1.0 |
| **Water** (DI) | | | 80.87 |
| | | **Total** | 100.00 |

| Base | | | |
|---|---|---|---|
| **Material** | **% Active** | **%w/w** | **Amount (g)** |
| EDTA (ex Sigma) | 100 | 0.6 | 0.6 |
| Sodium Sulphite (ex Fisher) | 100 | 1.0 | 1.0 |

(continued)

| Base | | | |
|---|---|---|---|
| **Material** | **% Active** | **%w/w** | **Amount (g)** |
| Sodium Isoascorbate (ex Lowenstein) | 100 | 0.15 | 0.15 |
| Propylene Glycol (ex Sigma) | 100 | 8.4 | 8.4 |
| Lowenol C-243 (ex Lowenstein) | 100 | 8.0 | 8.0 |
| Lowenol S-216X (ex Lowenstein) | 100 | 22.2 | 22.2 |
| Oleic Acid (ex Aldrich) | 100 | 8.6 | 8.6 |
| Isopropanol (ex Sigma) | 100 | 12.5 | 12.5 |
| Cholesterol (ex Sigma) | 100 | 0.3 | 0.3 |
| Ammonia (ex Sigma) | 28 | 33 | 33 |
| Water (DI) | | | 5.25 |
| | | **Total** | 100.00 |

## Example 3

## Hair Colouring Composition

A hair colouring composition was prepared in conventional manner according to the formulation details below. The developer and base were stored in separate bottles before use. To use, the developer and base are mixed together to form a hair colouring composition having a pH greater than pH10. The composition is applied to hair and left for 20 minutes. The hair is coloured effectively with minimal damage.

| Developer | | | |
|---|---|---|---|
| **Material** | **% AD** | **% w/w** | **Amount (g)** |
| Lowenol 6559 (ex Lowenstein) | 100 | 1.0 | 1.0 |
| Phosphoric Acid (ex Sigma) | 85 | 0.03 | 0.03 |
| Hydrogen Peroxide (ex Fisher) | 35 | 17.1 | 17.1 |
| Polyquaternium 37 (ex Ciba) | 100 | 1.0 | 1.0 |
| **Water** (DI) | | | 80.87 |
| | | **Total** | 100.00 |

| Dye Base | | | |
|---|---|---|---|
| Material | % AD | % w/w | Amount (g) |
| EDTA (ex Sigma) | 100 | 0.6 | 0.6 |
| Sodium Sulphite (ex Fisher) | 100 | 1.0 | 1.0 |
| Sodium Isoascorbate (ex Lowenstein) | 100 | 0.15 | 0.15 |
| Propylene Glycol (ex Sigma) | 100 | 8.4 | 8.4 |
| Lowenol C-243 (ex Lowenstein) | 100 | 8.0 | 8.0 . |
| Lowenol S-216X (ex Lowenstein) | 100 | 22.2 | 22.2 |
| Oleic acid (ex Aldrich) | 100 | 8.6 | 8.6 |
| Isopropanol (ex Sigma) | 100 | 12.5 | 12.5 |
| Cholesterol (ex Sigma) | 100 | 0.3 | 0.3 |
| Ammonia (ex Sigma) | 28 | 33.0 | 33.0 |
| RODOL DJ p-phenylene-diamine (Primary Intermediate) (ex Lowenstein) | 100 100 | 1.0 1.0 | 1.0 1.0 3.25 |
| RODOL RS resonanol (Coupler) (ex Lowenstein) | | | |
| **Water** (DI) | | | |
| | | **Total** | 100.00 |

**Claims**

1. A hair bleaching composition comprising:

   (a) a peroxygen compound;
   (b) a buffering agent; and
   (c) cholesterol and/or derivatives thereof or mixtures thereof;

   **characterised in that** the pH of the composition is greater than or equal to pH 10.3.

2. A hair bleaching composition according to claim 1 wherein the pH of the composition is greater than or equal to pH 10.5.

3. A hair bleaching composition according to any preceding claim wherein the peroxygen compound is hydrogen peroxide.

4. A hair bleaching composition according to any preceding claim wherein the buffering agent is ammonia.

5. A hair bleaching composition according to any preceding claim further comprising at least one surfactant.

6. A method of bleaching hair comprising applying to the hair a composition according to any of claims 1 to 5.

7. A hair colouring composition comprising the hair bleaching composition according to any of claims 1 to 5 and additionally a hair colouring agent.

8. A method of colouring hair comprising applying to the hair a composition according to claim 7.

9. A hair bleaching kit comprising:

   (a) a first package containing a peroxygen compound;
   (b) a second package containing a buffering agent;
   (c) cholesterol and/or derivatives thereof or mixtures thereof in the first and/or second package and/or in a third package;

   **characterised in that** the peroxyen compound, buffering agent and cholesterol and/or derivatives thereof or mixtures thereof form, when mixed, a hair bleaching composition according to claim 1 having a pH greater than or equal to 10.3.

10. A method of bleaching hair using a hair bleaching kit according to claim 9 comprising admixing the contents of the packages followed by applying the composition to the hair.

11. A hair colouring kit comprising:

   (a) a first package containing a water soluble peroxygen compound;

   (b) a second package containing a buffering agent;

   (c) cholesterol and/or derivatives thereof or mixtures thereof in the first and/or second package and/or in a third package;

   (d) a hair colouring agent contained in the second package and/or third package and/or a fourth package;

   **characterised in that** the water soluble peroxygen compound, buffering agent, hair colouring agent and cholesterol and/or derivatives thereof or mixtures thereof form, when mixed, a hair colouring composition according to claim 7 having a pH greater than or equal to 10.3.

12. Method of colouring hair using a hair colouring kit according to claim 11 comprising admixing the contents of the packages followed by applying the composition to the hair.

**13.** Method of reducing damage to hair, protecting hair from damage and/or improving damage repair of hair treated with bleaching and/or colouring compositions buffered to a pH greater than 10 comprising adding cholesterol to the bleaching and/or colouring composition.

**14.** Use of cholesterol to reduce damage to hair, protect hair from damage and/or improve damage repair of hair treated with bleaching and/or colouring compositions buffered to a pH of greater than 10.


**Patentansprüche**

**1.** Haarbleichende Zusammensetzung, umfassend:

a) eine Persauerstoff-Verbindung;
b) ein Puffermittel; und
c) Cholesterin und/oder Derivate davon oder Gemische davon;

**dadurch gekennzeichnet, dass** der pH-Wert der Zusammensetzung größer als oder gleich pH 10,3 ist.

**2.** Haarbleichende Zusammensetzung nach Anspruch 1, worin der pH-Wert der Zusammensetzung größer als oder gleich 10,5 ist.

**3.** Haarbleichende Zusammensetzung nach einem beliebigen vorangehenden Anspruch, worin die Persauerstoff-Verbindung Wasserstoffperoxid ist.

**4.** Haarbleichende Zusammensetzung nach einem beliebigen vorangehenden Anspruch, worin das Puffermittel Ammoniak ist.

**5.** Haarbleichende Zusammensetzung nach einem beliebigen vorangehenden Anspruch, die weiterhin mindestens ein Tensid umfasst.

**6.** Verfahren zum Bleichen von Haar, umfassend Auftragen einer Zusammensetzung nach einem beliebigen der Ansprüche 1 bis 5 auf das Haar.

**7.** Haarfärbende Zusammensetzung, umfassend die haarbleichende Zusammensetzung nach einem beliebigen der Ansprüche 1 bis 5 und zusätzlich ein Haarfärbemittel.

**8.** Verfahren zum Färben von Haar, umfassend Auftragen einer Zusammensetzung nach Anspruch 7 auf das Haar.

**9.** Haarbleichendes Kit, umfassend:

a) eine erste Packung, die eine Persauerstoff-Verbindung enthält;
b) eine zweite Packung, die ein Puffermittel enthält;
c) Cholesterin und/oder Derivate davon oder Gemische davon in der ersten und/oder zweiten Packung und/oder in einer dritten Packung;

**dadurch gekennzeichnet, dass** die Persauerstoff-Verbindung, das Puffermittel und das Cholesterin und/oder Derivate davon oder Gemische davon, wenn vermischt, eine haarbleichende Zusammensetzung nach Anspruch 1 mit einem pH-Wert größer als oder gleich 10,3 bilden.

**10.** Verfahren zum Bleichen von Haar unter Verwendung eines haarbleichenden Kits nach Anspruch 9, umfassend Anmischen des Inhalts der Packungen, gefolgt von Auftragen der Zusammensetzung auf das Haar.

**11.** Haarfärbendes Kit, umfassend:

a) eine erste Packung, die eine in Wasser lösliche Persauerstoff-Verbindung enthält;
b) eine zweite Packung, die ein Puffermittel enthält;
c) Cholesterin und/oder Derivate davon oder Gemische davon in der ersten und/oder in der zweiten Packung und/oder in einer dritten Packung;

## EP 1 237 530 B1

d) ein Haarfärbemittel, das in der zweiten Packung und/oder dritten Packung und/oder einer vierten Packung enthalten ist;

**dadurch gekennzeichnet, dass** die in Wasser lösliche Persauerstoff-Verbindung, das Puffermittel, das Haarfärbemittel, das Cholesterin und/oder Derivate davon oder Gemische davon, wenn vermischt, eine haarfärbende Zusammensetzung nach Anspruch 7 mit einem pH-Wert größer als oder gleich 10,3 bilden.

12. Verfahren zum Färben von Haar unter Verwendung eines haarfärbenden Kits nach Anspruch 11, umfassend Anmischen des Inhalts der Packungen, gefolgt von Auftragen der Zusammensetzung auf das Haar.

13. Verfahren zum Vermindern der Schädigung von Haar, Schützen von Haar vor Schädigung und/oder Verbesserung der Reparatur von Schädigung von Haar, das mit bleichenden und/oder färbenden Zusammensetzungen, gepuffert zu einem pH-Wert größer als 10, behandelt wurde, umfassend Zugeben von Cholesterin zu der bleichenden und/oder färbenden Zusammensetzung.

14. Verwendung von Cholesterin zur Verminderung von Schädigung für Haar, Schutz des Haares vor Schädigung und/oder Verbesserung der Reparatur von Schädigung von Haar, das mit bleichenden und/oder färbenden Zusammensetzungen, gepuffert auf einen pH-Wert größer als 10, behandelt wurde.

### Revendications

1. Composition de décoloration des cheveux comprenant :

   (a) un composé peroxygène ;
   (b) un agent tampon ; et
   (c) du cholestérol et/ou des dérivés de celui-ci ou des mélanges de celui-ci ;

   **caractérisé en ce que** le pH de la composition est supérieur ou égal au pH 10,3.

2. Composition de décoloration des cheveux selon la revendication 1, dans laquelle le pH de la composition est supérieur ou égal au pH 10,5.,

3. Composition de décoloration des cheveux selon l'une quelconque des revendications précédentes, dans laquelle le composé peroxygène est du peroxyde d'hydrogène.

4. Composition de décoloration des cheveux selon l'une quelconque des revendications précédentes, dans laquelle l'agent tampon est de l'ammoniaque.

5. Composition de décoloration des cheveux selon l'une quelconque des revendications précédentes, comprenant en outre au moins un agent tensioactif.

6. Procédé de décoloration des cheveux comprenant l'application aux cheveux d'une composition selon l'une quelconque des revendications 1 à 5.

7. Composition de coloration des cheveux comprenant la composition de décoloration des cheveux selon l'une quelconque des revendications 1 à 5 et de plus un agent de coloration des cheveux.

8. Procédé de coloration des cheveux comprenant l'application aux cheveux d'une composition selon la revendication 7.

9. Nécessaire de décoloration des cheveux comprenant :

   (a) un premier emballage contenant un composé peroxygène ;
   (b) un deuxième emballage contenant un agent tampon ;
   (c) du cholestérol et/ou des dérivés de celui-ci ou des mélanges de celui-ci dans le premier et/ou le deuxième emballage et/ou dans un troisième emballage ;

**caractérisé en ce que** le composé peroxygène, l'agent tampon et le cholestérol et/ou les dérivés de celui-ci ou les mélanges de celui-ci forment, lorsqu'ils sont mélangés, une composition de décoloration des cheveux selon la revendication 1 ayant un pH supérieur ou égal à 10,3.

10. Procédé de décoloration des cheveux utilisant un nécessaire de décoloration des cheveux selon la revendication 9, comprenant le mélange des contenus des emballages suivi par l'application de la composition aux cheveux.

11. Nécessaire de coloration des cheveux comprenant :

(a) un premier emballage contenant un composé peroxygène soluble dans l'eau ;
(b) un deuxième emballage contenant un agent tampon ;
(c) du cholestérol et/ou des dérivés de celui-ci ou des mélanges de celui-ci dans le premier et/ou le deuxième emballage et/ou dans un troisième emballage ;
(d) un agent de coloration des cheveux contenu dans le deuxième emballage et/ou le troisième emballage et/ou un quatrième emballage ;

**caractérisé en ce que** le composé peroxygène soluble dans l'eau, l'agent tampon, l'agent de coloration des cheveux et le cholestérol et/ou les dérivés de celui-ci ou les mélanges de celui-ci forment, lorsqu'ils sont mélangés, une composition de décoloration des cheveux selon la revendication 7 ayant un pH supérieur ou égal à 10,3.

12. Procédé de coloration des cheveux utilisant un nécessaire de coloration des cheveux selon la revendication 11, comprenant le mélange des contenus des emballages suivi par l'application de la composition aux cheveux.

13. Procédé de réduction de l'endommagement des cheveux, de protection des cheveux contre un endommagement et/ou d'amélioration de la réparation de l'endommagement des cheveux traités par des compositions de décoloration et/ou de coloration tamponnées à un pH supérieur à 10 comprenant l'addition de cholestérol à la composition de décoloration et/ou de coloration.

14. Utilisation de cholestérol pour réduire l'endommagement des cheveux, pour protéger les cheveux contre un endommagement et/ou pour améliorer la réparation de l'endommagement des cheveux traités par des compositions de décoloration et/ou de coloration tamponnées à un pH supérieur à 10.